Europäisches Patentamt

European Patent Office

Office européen des brevets

(11) Publication number : **0 328 389 B1**

# EUROPEAN PATENT SPECIFICATION

(45) Date of publication of patent specification :
**01.07.92 Bulletin 92/27**

(51) Int. Cl.⁵ : **A61K 47/00, A61K 31/475,**
**A61K 37/12**

(21) Application number : **89301258.3**

(22) Date of filing : **09.02.89**

(54) Compostions for treating intracranial tumors.

(30) Priority : **11.02.88 US 154984**

(43) Date of publication of application :
**16.08.89 Bulletin 89/33**

(45) Publication of the grant of the patent :
**01.07.92 Bulletin 92/27**

(84) Designated Contracting States :
**AT BE CH DE ES FR GB GR IT LI LU NL SE**

(56) References cited :
**EP-A- 0 167 263**
**DE-A- 2 844 534**

(56) References cited :
**Biological abstracts, abstracts n 35040228,
RRM R. Sutton et al "Matrix associated
chemotherapy for the localized treatmentof
experimental murine brain tumors" & 79th
annual meeting of the American Association
for Cancer Researc, New Orleans,Louisiana,
US, May 25-28 1988, & Proc. Am. Assoc. Can-
cer Res. 1988, vol. 29, n 0,page 507**

(73) Proprietor : **MATRIX PHARMACEUTICAL INC.
1430 O'Brien Drive Suite H
Menlo Park California 94025 (US)**

(72) Inventor : **Luck, Edward E.
216 Robin Way
Menlo Park California 94025 (US)**
Inventor : **Brown, Dennis M.
211 Robin Way
Menlo Park California 94025 (US)**

(74) Representative : **Harrison, David Christopher
et al
MEWBURN ELLIS & CO 2/3 Cursitor Street
London EC4A 1BQ (GB)**

## Description

The present invention relates to the making of compositions for treating intracranial tumors and surrounding tissues.

Cancer chemotherapeutics for treatment of brain cancer are limited. There are few systemically-delivered drugs which traverse the blood-brain barrier. Further, neurotoxicity of many of these drugs prevents providing a dose which is adequate to kill tumor cells in the brain without impairing normal brain cells.

With the exception of the nitrosoureas, few currently used anti-cancer agents possess the physiochemical properties to adequately penetrate the blood-brain barrier and consequently do not reach appreciable concentrations in the brain following systemic administration. While the nitrosoureas have been widely used for brain tumor chemotherapy, their effects on patient survival rates have been disappointing. This lack of effectiveness has been attributed to tumor cell heterogeneity, insensitivity of the tumor cells to the drug, and inadequate drug delivery to avascular quiescent cells.

A number of techniques have been devised to administer water soluble anti-cancer drugs across the blood-brain barrier. These techniques include intracerebrospinal fluid (CSF) chemotherapy, transient, reversible blood-brain barrier modification and utilization of carrier-mediated transport systems at the blood-brain barrier. Transient-reversible blood-brain barrier modification techniques include the osmotic opening technique and opening the blood-brain barrier using either metrazol or X-ray therapy. Carrier-mediated transport systems have been based on the use of D-glucose, L-amino acids, neurotransmitter precursors, and ions.

Greig, Cancer Treatment Reviews (1987) 14:1-28 describes optimizing drug delivery to brain tumors, and in particular, the circumvention of the blood-brain barrier at page 7. US-A-4,619,913 (EP-A-167263) describes treatments employing drug-containing matrices for introduction into cellular lesion areas.

In the present invention, we provide the use of a vinca alkaloid dispersed in a flowable proteinaceous matrix for the manufacture of an aqueos-medium flowable composition for treating intracranial abnormal solid cellular masses, particularly tumors or adjacent tissues that may contain tumor cells. The matrix substantially inhibits the migration of the drug from the site of administration, so as to maintain the primary effect of the drug in the region of administration. Migration can be further inhibited by the use, in the manufacture, of physiologically acceptable materials which modify cellular properties or physiological responses to further regionalize the placement of drug at the injection site.

Underlying the invention is the discovery that the neurotoxic effects of the vinca alkaloids can be sufficiently reduced to permit intracranial chemotherapy when those cytotoxic agents are administered with peptide and The use employs a substantially uniform dispersion of a vinca alkaloid drug in a concentrated dispersion of a physiologically acceptable matrix, particularly a protein such as collagen, fibrinogen, or a derivative thereof, dispersed in a minor amount of a physiologically acceptable aqueous medium. The resulting amorphous mass is administered, for example, by injection into the tumor or lesion area, e.g., adjacent tissue or, in those situations where the tumor has been removed, tissue adjacent to the previously removed tumor.

The proteinaceous matrix is flowable for administration as by injection or packing into a lesion area following tumor removal, but provides for stable placement once administered. That is, the proteinaceous matrix remains substantially where introduced and does not migrate significantly.

In addition to the matrix material will be a vinca alkaloid drug individually or in combination with one or more other chemotherapeutic agents and a physiologically acceptable aqueous medium in which the proteinaceous composition is dispersed and the drug(s) may be dissolved or dispersed in the aqueous medium. Other materials are preferably present to enhance the beneficial properties of the subject composition.

The treatment may be employed with various solid tumors including gliomas, astrocytomas, carcinomas and sarcomas. After administration, the vinca alkaloid is slowly released into the immediate environment so as to maintain a high localized cellular uptake. Thus, substantial transportation of the drug to other sites where its cytotoxic effect is undesirable is prevented. The level of the drug remains low in areas of the brain distant from the administration site. In this way, a therapeutic dose of a vinca alkaloid can be provided to the tumor area, while minimizing the neurotoxic effects on other parts of the brain.

Illustrative of the various disease states or therapeutic modes in which the subject invention may find application are: (1) intracranial neoplasms in which local recurrence is typical and drug bioavailability is compromised; (2) intracranial tumors which are poor candidates for surgical or radiation management; (3) adjunctive tumor therapy in combination with physical or non-chemical treatments, e.g., radiation and/or hyperthermia.

The vinca alkaloids, including vinblastine and vincristine, produce a stathmokinetic effect. The mechanism of action involves binding to tubulin, a dimeric protein involved in cell functions including mitosis, transport of solutes and cell movement, and interference with the metabolic pathways of amino acids leading from glutamic acid through the citric acid cycle to urea. Vinblastine and vincristine are indicated for the treatment of many non-central nervous system (CNS) tumors.

The vinca alkaloids have been contra-indicated in CNS treatment because of their neurotoxic effects. With the vinca alkaloids, the dose-limiting clinical toxicity is generally manifested as neurotoxicity. Vinca alkaloids, when used in large dosages for long periods, have led to convulsions, severe and permanent central nervous system damage and even death. In particular, vinblastine sulfate has been associated with numbness, paresthesias, peripheral neuritis, mental depression, loss of deep-tendon reflexes, headache, convulsions, and severe central nervous damage and death. Vincristine sulfate has been associated with paresthesia, neuritic pain, loss of deep-tendon reflexes, paralysis, and convulsions. The vinca alkaloids are also known to not penetrate well into cerebral spinal fluid. Thus, the vinca alkaloids have not found acceptance in the treatment of brain tumors.

It has now been found that when administered as a proteinaceous composition, an effective amount of a vinca alkaloid can be delivered to the tumor site without untoward neurotoxic effects distant from the site. The subject compositions are amorphous, injectable and viscous, so as to substantially retain a localized position without significant flow from the site of administration. The compositions can flow under moderate pressure, but will not move significantly after being positioned at a particular site. Preferably, a substantial proportion of the composition will be comprised of the matrix to provide the desired composition characteristics. The matrix may be comprised of individual or combinations of peptides or proteins such as collagen and fibrinogen or derivatives thereof.

Proteinaceous compositions having at least about 5 weight percent, preferably at least about 10 weight percent, and up to 50 weight percent or more, are of particular interest when used in combination with thrombin or its enzymatic equivalent. In this way, fibrinogen is enzymatically modified to fibrin to enhance the non-migratory property of the composition while forming a matrix of fibrils to further stabilize the composition.

The thrombin may be mixed with the fibrinogen containing proteinaceous composition from a time immediately prior to use or shortly after injection. The amount of thrombin of about 1 to 1000 IU/mg employed will generally range from about 0.1 to 10 weight percent of the fibrinogen present, depending upon the time of use, the rate desired for solid matrix formation, the amount of other components, the effect of the drug on thrombin activity, and the like.

The collagenous or fibrinogen-containing material which is used may be derived from any mammalian host source such as bovine, porcine or human, or may be natural collagen or may be modified, such as tropocollagen, atropocollagen, or the like. The collagen may be non-immunogenic, immunogenic, or only slightly immunogenic.

Various methods for preparing collagen or derivatives thereof in purified form for administration to a mammalian host are known in the literature. These methods may be found in such patents as U.S. Patent No. 3,949,073 and references cited therein. Of interest is bovine collagen which is purified and is obtained from young cows or calves. Purification will normally involve dispersion or precipitation from various media, e.g., dilute acetic acid. In some situations, xenogeneic collagen is employed to enhance an immunogenic response in the area of injection or immunogenic adjuvants may be employed.

Various chemotherapeutic drugs may be employed in conjunction with a vinca alkaloid depending upon the nature of the additional drug, the tumor, and whether cooperative action is pharmacologically indicated. Drugs which are used in chemotherapy may act as alkylating agents, enzyme inhibitors, proliferation inhibitors, lytic agents, DNA synthesis inhibitors, membrane permeability modifiers, DNA intercalators, antimetabolites, or the like. Illustrative drugs include chlorambucil, melphalan, busulfan, carmustine, lomustine, streptozotocin, thiotepa, dicarbazine, methotrexate, 5-fluorouracil, cytarabine, azaribine mercaptopurine, thioguanine, actinomycin D, adriamycin, bleomycin, mithramycin, mitomycin C, L-asparaginase, cisplatin, procarbazine, prednisone, prednisilone, triamicinolone, testosterone, estrogen, insulins, and hydroxyurea. See Carter and Livingston, Drugs Available to Treat Cancer, In Principles of Cancer Treatment (Carter et al, eds.) Chapter 10, pp. 111-145, 1982, McGraw-Hill, Inc., N.Y. The drugs should not form non-enzymatically-labile bonds with the matrix material resulting in the loss of their therapeutic effect.

Other drugs for use in combination with the chemotherapeutic agents are drugs which retard the diffusion away of the chemotherapeutic agent, so as to further reduce physiological insult and enhance therapeutic gain. Of particular interest are agents which restrict blood flow in the tumor or the regional vasculature, e.g., vasoconstrictive or sympathomimetic agents. These agents may include catecholamines, e.g., epinephrine and norepinephrine, ergot alkaloids, prostaglandins and angiotensin. Calcium ion agonists or calcium ion influx inhibitors, such as verapamil, also find use to further retard diffusion of the chemotherapeutic agent(s) from the area of administration.

Besides using xenogeneic collagen, other materials may be included to enhance an immunogenic response, e.g., proliferation and invasion of macrophage, helper T-cells, etc. Illustrative adjuvants include Corynebacterium parvum, Bacillus Calmette-Guerin cell wall or cell wall skeleton preparations, Mycobacterium bovis strain, etc. See Miyata et al, Cancer Res. (1983) 43:4670-4675; Bier et al, Arch. Otorhinolaryngol. (1982) 236:245-255; and Mehanjhlin et al, Cancer Res. (1978) 38:1311-1316, whose relevant disclosure is incorporated herein by reference.

For enhancing cytotoxic activity, various adjuvant materials may be incorporated into the matrix such as radioactive pellets, e.g., radionuclides Technicium or Iridium; radiation sensitizers, e.g., misonidazole and bromodeoxyuridine; repair inhibitors, e.g., methylated xanthines; bioreductive agents which are activated only in hypoxic cells, e.g., mitomycin C and SR-4233; immunomodifiers such as interferons, lymphokines such as interleukin-2; tumor growth inhibitors such as tumor necrosis factor, tumor growth factor-β, etc., and/or angiographic contrast media.

As already indicated, the ratio of dry materials in the composition may vary widely. However, the amount of protein matrix material will usually be not less than 30% and not greater than about 95%, generally ranging from about from 40% to 90%, more usually ranging from about 50% to 90% by weight. Of this, preferably 10% to 100% will be collagen and/or fibrinogen. The chemotherapeutic drug(s) will normally be a liquid or solid, or provided in solid form and will generally range from at least about 0.1% by weight to up to about 50% by weight, more usually being from about 1% to 50% by weight, generally being from about 1% to 45% by weight of the proteinaceous material.

Other ancillary additives or agents will vary in total amount from about 0.005 to 15, usually from about 0.01 to 10 weight percent of the dry weight of the total composition.

The composition is uniformly dispersed in a physiologically acceptable aqueous medium such as saline, phosphate buffered saline, distilled water, etc. The aqueous medium will be sufficient to provide for an amorphous dispersion capable of flowing under mild pressure. Usually, the liquid aqueous medium will be at least 90 weight percent of the entire composition, more usually at least 95 weight percent and not more than about 99.8 weight percent, usually not more than about 99.5 weight percent, so as to provide a flowable mixture. The amount will vary depending upon the nature of the drug(s), the nature of the matrix material, the presence of other materials, and the like. The concentration of protein in the aqueous medium will range from about 2 to 75 mg/ml.

In addition to the major components, a number of minor components may also be included for a variety of purposes. These agents will, for the most part, impart properties which protect the stability of the composition, control the pH, or the like. Illustrative agents include phosphate or acetate buffers, methyl or propyl paraben, polyethylene glycols, etc. These agents generally will be present in less than about 2 weight percent of the total composition, usually less than about 1 weight percent, and individually may vary from about 0.001 weight percent to about 1 weight percent.

The composition can be prepared by combining the various components in a sterile environment. The matrix will be provided in a convenient form, usually admixed with at least a portion of the total aqueous medium to be employed. The composition will be sufficiently workable that upon admixture of the other agents, a uniform dispersion can be obtained. When collagen or derivative thereof is used, the collagenous material will normally be provided as a uniform dispersion of collagen fibrils in an aqueous medium, where the collagenous material will be from about 5 mg/ml to not more than 100, usually not more than 75 mg/ml. The drug may then be added to the collagenous dispersion with agitation to ensure the uniform dispersion of the drug in the resulting mixture. Other materials, as appropriate, may be added concomitantly or sequentially. After ensuring the uniform dispersion of the various components in the mixture, the mixture may be sterilized or the components of the mixture may be sterilized prior to mixing. The sterile mixture will be sealed in an appropriate container. Sterilization will usually be achieved using aseptic conditions.

The subject composition can be used in the treatment of a wide variety of intracranial neoplastic lesions where the lesions are primary or secondary tumors of various origins.

The subject composition will be administered to an intracranial tumor to provide a cytotoxic amount of drug at the tumor site. The amount of cytotoxic drug(s) administered to the tumor site will generally range from about 0.1 to 500, more usually from about 0.1 to about 50, most usually about 0.5 to 30 mg/kg of host, depending upon the nature of the drug(s), size of tumor, and other considerations. When a vinca alkaloid is the sole chemotherapeutic agent in the composition, the concentration will range from about 0.05 to about 10 mg/kg of host, usually about 0.1 to about 5.0 mg/kg, more usually about 0.5 to about 2.0 mg/kg. The vasoconstrictive agents will generally be present in from 1 to 50 weight percent of the therapeutic agent. In view of the wide diversity of tumors, nature of tumors, effective concentrations of vinca alkaloids when used with other agents, relative mobility and the like, a definitive range cannot be specified. With each drug combination in each tumor, experience will provide an optimum level. One or more administrations may be employed, depending upon the lifetime of the drug at the tumor site and the response of the tumor to the drug. Administration may be by syringe, catheter, needle or other convenient means allowing for introduction of a flowable composition into the tumor either directly or sterotactically inserted through a burr hole for non-resectable tumors. Administration may be every three days, weekly, or less frequent such as biweekly or at monthly intervals. Administration may require diagnostic imaging and the use of sterotactic frames to insure precise needle or catheter placement.

Illustrative of the manner of administration according to this invention would be administration of vinblastine. Drug concentrations in the matrix may vary from about 0.01 to about 4 mg/ml. Injection into a tumor which will not be removed or adjacent tissue may be at one or more sites depending on the size of the lesion. Needles of about 1-2mm diameter are convenient intraoperatively. When the tumor is removed, the composition may be packed into the resulting lesion or applied to the tissue adjacent the former location of the tumor. The drug dose will normally be less than 3 mg/kg of patient.

The subject method is also found to reduce local inflammation as a result of the drug administration. Therefore, local or adjacent tissue is less likely to be affected by the drug. Furthermore, due to the low migratory level of the drug from the site of placement, higher drug dosages can be administered to the site without adverse affects to normal tissue distant from the placement site.

The following examples are provided by way of illustration and not by way of limitation.

## EXPERIMENTAL

## Example 1

The use of vinblastine (VBL) and vincristine (VC) alone and in combination with a protein carrier on the growth of experimental murine tumors grown intracranially was investigated. Halothane-anesthesized C3H mice 12-16 weeks in age were injected intracranially (i.c.) into the right frontal lobe with 10 µl total volume of Hank's balanced salt solution containing $5 \times 10^4$ KHT fibrosarcoma cells through a burr hole made in the exposed cranium with a compressed air-driven drill. Following injection, the cranium was sealed with bone wax, and the overlying skin was sutured. Five days post tumor implantation the mice were randomly divided into the following groups based on the treatment solution administered intracranially:

saline controls

vinblastine (0.2 mg/kg)

vinblastine + epinephrine (0.1 mg/kg)

vinblastine + matrix (1:1 bovine collagen 36 mg/ml)

vinblastine + epinephrine (0.1 mg/kg) + matrix (1:1 bovine collagen 36 mg/ml)

The test solution with and without matrix and the saline control were delivered intracranially (i.c.) in an injection volume of 10 µl through the same burr hole used for tumor cell inoculation. All mice were observed daily.

The acute drug toxicity level was determined by the percentage of animal survival on day 7 following drug injection. A comparison of the acute toxicity (deaths) of mice treated i.c. with matrix-associated VBL to aqueous VBL (10 µl) injection volume to the right frontal lobe is shown in Table 1 below. Deaths were attributed to drug toxicity since histopathological evaluation of the mice at autopsy showed no evidence of KHT brain tumor.

5

TABLE I

Evaluation of Acute Toxicity
to Intracerebrally Implanted
Matrix Associated Vinblastine

|  | Treatment | Number of Mice Per Group | Number of Mice That Died | % of Mice That Died |
|---|---|---|---|---|
| Group 1 | .2 mg/kg Vinblastine | 27 | 7 | 26 |
| Group 2 | .2 mg/kg Vinblastine + Matrix | 24 | 2 | 8 |
| Group 3 | .2 mg/kg Vinblastine + Epi | 28 | 9 | 32 |
| Group 4 | .2 mg/kg Vinblastine + Matrix + Epi | 25 | 1 | 4 |
| Group 5 | Saline | 26 | 0 | 0 |

At the dose level studied (0.2 mg/kg), matrix-associated vinblastine was shown to be significantly less neurotoxic. In particular, it was observed that a greater percentage of mice were alive on Day 7 for mice treated with matrix-associated vinblastine, with and without epinephrine, Groups 4 and 2, respectively, (4-8% of mice died) in comparison to the i.c. administration of the aqueous form of VBL Groups 1 and 3 (about 30% of mice died).

Surviving mice from each group were sacrificed on day 16 or 18 following tumor implantation. Cross-sections of whole mouse brains were examined histopathologically for the presence of tumor involvement in the brain. The results are shown in Table II.

## TABLE II

### Histopathological Evaluation of KHT Brain Tumor Response to Intracerebrally Implanted Matrix Associated Vinblastine

| | Treatment | Number of Mice Per Group | Large[1] | Number of Mice With Varying Tumor Size | | |
| --- | --- | --- | --- | --- | --- | --- |
| | | | | Small[2] | NED[3] | %NED |
| Group 1 | .2 mg/kg Vinblastine | 12 | 2 | 7 | 3 | 25 |
| Group 2 | .2 mg/kg Vinblastine + Matrix | 17 | 2 | 3 | 12 | 71 |
| Group 3 | .2 mg/kg Vinblastine + Epi | 13 | 3 | 5 | 5 | 39 |
| Group 4 | .2 mg/kg Vinblastine + Matrix + Epi | 18 | 2 | 2 | 14 | 78 |
| Group 5 | Saline | 19 | 10 | 7 | 2 | 11 |

[1] Large tumor = a tumor that comprises 30% or more of the cross-sectional area of the histopathological microscopic field of C3H whole brains observed from mice sacrificed on days 16 and 18 post treatment

[2] Small tumor = a tumor that comprises 5-30% of the cross-sectional area of the histopathological microscopic field of C3H whole mice brains observed from mice sacrificed on days 16 and 18 post treatment

[3] NED = No evidence of disease, i.e. no tumor observed in a cross-sectional area of the histopathological microscopic field of C3H whole mice brains observed from mice sacrificed on days 16 and 18 post treatment

As shown in Table II, approximately 71-78% of mice treated with matrix-associated VBL (Groups 2 and 4) were without observable tumor (NED), while only 25% and 39% of mice treated with aqueous VBL (Groups 1 and 3) were NED. Saline-injected control mice (Group 5) had approximately 11% NED. Therefore, matrixassociated VBL was more effective against the KHT brain tumor and less toxic to the C3H mice than VBL alone.

The data demonstrates that the neurotoxic effects of vinca alkaloids are significantly reduced by administering the drugs in a stable, flowable proteinaceous matrix.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced.

## Claims

1. Use of a uniform dispersion of a vinca alkaloid and a physiologically acceptable macro-molecular proteinaceous matrix material for the manufacture of an aqueous-medium flowable composition for use in treating an intracranial neoplastic lesion or surrounding tissue.

2. A use according to claim 1 wherein the macromolecular matrix is at least in part collagen.

3. A use according to claim 1 wherein the macromolecular matrix is at least in part fibrinogen.

4. A use according to claim 1, claim 2 or claim 3 wherein the vinca alkaloid drug is vinblastine.

5. A use according to claim 1, claim 2 or claim 3 wherein the vinca alkaloid drug is vincristine.

6. A use according to any one of the preceding claims which additionally comprises the use for the composition of a sufficient amount of a vasoconstrictor to constrict capillaries in the vicinity of a lesion.

7. A use according to claim 6 wherein the vasoconstrictor is epinephrine or non-epinephrine.

8. A use according to any one of the preceding claims which wherein the matrix comprises from about 30% to about 95% of collagen and/or fibrinogen dispersed in an aqueous medium at a concentration of from about 2 to about 75 mg/ml and the vinca alkaloid is used in an amount of from 0.1 to 50 weight percent, based on collagen.

## Patentansprüche

1. Verwendung einer gleichmäßigen Dispersion eines Vincaalkaloids und eines physiologisch verträglichen makromolekularen Matrixmaterials auf Eiweißbasis zur Herstellung einer fließfähigen Zusammensetzung in wässerigem Medium zum Einsatz bei der Behandlung einer intrakranialen neoplastischen Läsion oder des umgebenden Gewebes.

2. Verwendung nach Anspruch 1, worin die makromolekulare Matrix zumindest zum Teil Kollagen ist.

3. Verwendung nach Anspruch 1, worin die makromolekulare Matrix zumindest zum Teil Fibrinogen ist.

4. Verwendung nach Anspruch 1, 2 oder 3, worin der Vincaalkaloid-Arzneimittelwirkstoff Vinblastin ist.

5. Verwendung nach Anspruch 1, 2 oder 3, worin der Vincaalkaloid-Arzneimittelwirkstoff Vincristin ist.

6. Verwendung nach einem der vorhergehenden Ansprüche, die zusätzlich den Einsatz einer ausreichenden Menge eines Vasokonstriktors für die Zusammensetzung umfaßt, um Kapillaren in der Nachbarschaft einer Läsion zu verengen.

7. Verwendung nach Anspruch 6, worin der Vasokonstriktor Epinephrin oder non-Epinephrin ist.

8. Verwendung nach einem der vorhergehenden Ansprüche, worin die Matrix von etwa 30 % bis etwa 95 % Kollagen und/oder Fibrinogen umfaßt, das in einem wässerigen Medium mit einer Konzentration von etwa 2 bis etwa 75 mg/ml dispergiert ist, und das Vincaalkaloid in einer Menge von 0,1 bis 50 Gew.-%, bezogen auf Kollagen, eingesetzt ist.

## Revendications

1. Utilisation d'une dispersion uniforme d'un alcaloïde de vinca et d'une matrice protéinée macromoléculaire physiologiquement acceptable pour la fabrication d'une composition fluide en milieu aqueux à utiliser pour le traitement d'une lésion néoplastique intracrânienne ou du tissu l'entourant.

2. Utilisation selon la revendication 1 où la matrice macromoléculaire est au moins en partie du collagène.

3. Utilisation selon la revendication 1 où la matrice macromoléculaire est au moins en partie du fibrinogène.

4. Utilisation selon la revendication 1, la revendication 2 ou la revendication 3 où l'alcaloïde de vinca comme médicament est la vinblastine.

5. Utilisation selon la revendication 1, la revendication 2 ou la revendication 3 où l'alcaloïde de vinca comme médicament est la vincristine.

6. Utilisation selon l'une quelconque des revendications précédentes qui comprend de plus l'utilisation, pour la composition, d'une quantité suffisante d'un vasoconstricteur pour resserrer les capillaires à proximité d'une lésion.

7. Utilisation selon la revendication 6 où le vasoconstricteur est l'épinéphrine ou la non-épinéphrine.

8. Utilisation selon l'une quelconque des revendications précédentes du type où la matrice contient environ 30% à environ 95% de collagène et/ou de fibrinogène en dispersion dans un milieu aqueux à une concentration comprise entre environ 2 et environ 75 mg/ml et l'alcaloïde de vinca est utilisé en une quantité de 0,1 à 50% en poids, en se basant sur le collagène.